(19) Europäisches Patentamt / European Patent Office / Office européen des brevets

(11) **EP 1 597 539 B1**

(12) **EUROPÄISCHE PATENTSCHRIFT**

(45) Veröffentlichungstag und Bekanntmachung des Hinweises auf die Patenterteilung:
**06.10.2010   Patentblatt 2010/40**

(21) Anmeldenummer: **04710351.0**

(22) Anmeldetag: **12.02.2004**

(51) Int Cl.:
*G01B 11/25* (2006.01)      *A61B 1/00* (2006.01)

(86) Internationale Anmeldenummer:
**PCT/EP2004/001290**

(87) Internationale Veröffentlichungsnummer:
**WO 2004/076970 (10.09.2004 Gazette 2004/37)**

(54) **VERFAHREN UND OPTISCHES SYSTEM ZUR VERMESSUNG DER TOPOGRAPHIE EINES MESSOBJEKTS**

METHOD AND OPTICAL SYSTEM FOR MEASURING THE TOPOGRAPHY OF A TEST OBJECT

PROCEDE ET SYSTEME OPTIQUE PERMETTANT DE MESURER LA TOPOGRAPHIE D'UN OBJET DE MESURE

(84) Benannte Vertragsstaaten:
**DE FR GB IT**

(30) Priorität: **27.02.2003   DE 10308383**

(43) Veröffentlichungstag der Anmeldung:
**23.11.2005   Patentblatt 2005/47**

(73) Patentinhaber: **Storz Endoskop Produktions GmbH**
**78532 Tuttlingen (DE)**

(72) Erfinder: **KRATTIGER, Beat**
**CH-8222 Beringen (CH)**

(74) Vertreter: **Stamer, Harald**
**Jahnstrasse 7**
**35579 Wetzlar (DE)**

(56) Entgegenhaltungen:
**WO-A-93/03579      DE-A- 4 410 134**
**US-A- 4 986 262**

- **PATENT ABSTRACTS OF JAPAN vol. 2003, no. 01, 14. Januar 2003 (2003-01-14) -& JP 2002 257527 A (AVAL DATA CORP), 11. September 2002 (2002-09-11)**

EP 1 597 539 B1

**Beschreibung**

Die Erfindung betrifft ein Verfahren zur endoskopischen Vermessung der Topographie eines Meßobjekts mit den im Oberbegriff des Anspruchs 1 genannten Merkmalen und ein dazu geeignetes Endoskop mit den im Oberbegriff des Anspruchs 6 genannten Merkmalen.

Optische Vermessungssysteme, die durch das parallaktische Einbelichten von komplexen Mustern auf ein Objekt eine Vielzahl von Raumkoordinaten der Oberfläche eines Meßobjektes gewinnen, sind bekannt. Im allgemeinen wird dabei ein regelmäßiges Streifenmuster auf das Meßobjekt projiziert. Die Streifenlage wird mit einem Scanner oder einer Kamera abgetastet. Der Messung liegt eine Triangulation zugrunde, so daß die Projektions- und Bildaufnahmewinkel und der Abstand zwischen Kamera und Projektor bekannt sein müssen. Weil die Streifen im einzelnen nicht identifizierbar sind, entsteht eine Modulo-Unsicherheit bei der Raumkoordinatenbestimmung, die durch Mehrfachaufnahmen mit jeweils verschiedenen Streifenabständen gelöst werden muß.

Auch die Projektion von regelmäßigen Punktemustern, die durch Laserbeleuchtung eines diffraktiven optischen Elements erzeugt werden, ist in der Technik der automatischen Objekterkennung bekannt. In Machine Vision News, Vol.4, (1999) wird die Verschiebung der Elemente eines regelmäßigen Punktemusters bei der Projektion auf eine unebene Oberfläche dargestellt. Es wird der allgemeine Hinweis gegeben, daß damit neue Möglichkeiten der Objekterkennung eröffnet würden, ohne diese bezüglich der Indentifizierung der Punkte auf der Oberfläche genauer zu beschreiben.

Um Modulo-Unsicherheiten bei der optischen Vermessung räumlicher Strukturen auszuschließen, werden überwiegend eindeutig indentifizierbare Meßmarken verwendet, die auf dem Meßobjekt aufgebracht werden. Mit Hilfe von Bildaufnahme- und Bildauswertesystemen können diese Meßmarken individuell erkannt und vermessen werden.

Die Vermessung dreidimensionaler Strukturen oder von Flächen und Strecken ist auch in der endoskopischen Meßtechnik von Bedeutung. Bei medizinischen Anwendungen ist z.B. die minimalinvasive Form- und Volumenerfassung von Tumoren als Entscheidungshilfe für die optimale Behandlung oder zur Überwachung des Wachstumsprozesses wichtig. In der technischen Endoskopie kann z.B. aufgrund eines quantitativ festgestellten Materialfehlers eine Zerlegung der Einrichtung vermieden oder aufgeschoben werden bis ein kritisches Ausmaß festgestellt wird. Bei diesen Anwendungen ist es nicht möglich, auf dem Meßobjekt spezielle Meßmarken anzubringen.

Neben anderen Meßmethoden wurde daher auch in der messenden Endoskopie das Prinzip der Bildauswertung der Parallaxe, d.h. der scheinbaren Seitwärtsverschiebung eines einbelichteten Musters in Abhängigkeit von der Projektionsentfernung, angewendet. Bei einem von K. Armbruster und M. Scheffler, Institut für angewandte Forschung in der Automation, FH Reutlingen, beschriebenen 3D-Meßendoskop wird strukturiertes Licht, z.B. ein Linienmuster, in den zu vermessenden Objektraum projiziert und das Szenenbild mit einer Kamera aus einer zweiten Perspektive aufgenommen. Bei diesem Verfahren ist die Position der Lichtquelle in Bezug zur Kamera definiert, so daß mittels Triangulation der 3D-Koordinaten einer Objektoberfläche berechnet werden können. Ein Verfahren zur Aufnahme und Vermessung von Körpern ist von Armbruster K. im Tagungsband zum 3. Symposium Bildverarbeitung, Technische Akademie Esslingen, (1993) angegeben.

In US 5 150 254 A1 wird das Prinzip der Formerkennung durch Auswertung einer aufprojizierten Markierungslinie allgemein beschrieben. Als wesentliches Problem werden die Stabilisierung des Projektionswinkels und die Lage der Markierungslinie genannt, da diese entscheidend für die genaue Ermittlung des Abstands zum Meßobjekt sind. Der Projektionswinkel und der Bildaufnahmewinkel sind so gewählt, daß die Markierungslinie in der Bildmitte liegt.

Aus US 5 669 871 A1 ist ein Endoskop mit einem am distalen Ende angeordneten Beleuchtungssystem, einem CCD-Bildaufnahmesystem und einem Projektionssystem für eine Referenzlinie bekannt. Die optischen Achsen des Bildaufnahmesystems und des Projektionssystems haben einen definierten Abstand zueinander. Es wird eine einzige Referenzlinie auf die Objektebene projiziert, wobei das Projektionssystem Justiermittel enthält, um eine möglichst gerade Referenzlinie zu erzeugen. Bei zylindrisch gewölbten Objektflächen muß das Endoskop so ausgerichtet werden, daß die Referenzlinie parallel zur Zylinderachse verläuft. Darüber hinaus muß jeweils dafür gesorgt werden, daß die Referenzlinie auf der zu vermessenden Objektstelle liegt.

Durch Bildauswertung werden für die dem Objektbild überlagerte Referenzlinie die Raumkoordinaten einer Geraden berechnet, die dem Bild der projizierten Referenzlinie angenähert ist. Aus den Verformungen der Referenzlinie im Objektbild gegenüber der berechneten Geraden lassen sich die Raumkoordinaten von ausgewählten Objektpunkten bestimmen, die auf der Referenzgeraden liegen.

Aus DE 35 16 164 C2 ist ein System zur optischen Längenmessung mit einem Endoskop bekannt. Am distalen Ende des Endoskops sind ein Projektionssystem und ein Bildaufnahmesystem in einem festen Abstand zueinander angeordnet. Die optischen Achsen beider Systeme sind parallel zueinander ausgerichtet und die Öffnungswinkel des Projektionskegels und des Bildaufnahmekegels sind gleich. Durch das Projektionssystem wird z.B. eine Kreismarkierung auf das Objekt projiziert, die im Bild des Meßobjekts dargestellt wird. Der Abstand der Kreismarkierung vom obersten Punkt des Sichtfeldes stellt eine Bezugslänge dar, deren Größe unabhängig von der jeweiligen Objektentfernung ist, weil aufgrund der gleichen Kegelwinkel die parallaktische Verschiebung der Begrenzung des Sichtfeldes und der Lage der Kreismarkierung parallel zueinander verlaufen.

Mit diesem Verfahren ist nur eine Längenschätzung durch relativen Längenvergleich möglich. Die Genauigkeit hängt zudem davon ab, ob die Begrenzung des Sichtfeldes und die Projektion der Kreismarkierung in derselben Ebene liegen. Eine Höheninformation über das Meßobjekt kann nicht gewonnen werden.

Aus WO - A - 93/03579 ist ein System zum räumlichen Sehen für optische Inspektions- und Roboter-Anwendungen bekannt. Das System enthält vorzugsweise zwei Projektoren und eine elektronische Kamera, wobei die Projektoren zwei sich kreuzende Linienscharen auf ein Objekt projizieren. Aufgrund der Überlagerung der Linienscharen sind die Kreuzungspunkte intensitätsmäßig hervorgehoben, so daß ihre Lage im Bildfeld der Kamera durch Kantenauswertung bestimmt werden kann.

Die beiden Projektoren sind so angeordnet, daß sich die Projektions-Zentrumslinien beider Projektoren in einer Ebene schneiden, wodurch im Schnittpunkt ein virtuelles Projektionszentrum entsteht, dem im Abstand der Projektionsbrennweite eine virtuelle Projektionsebene mit den überlagerten Linienscharen zugeordnet ist. Die Verbindungslinie zwischen dem Projektionszentrum und dem Bildmittelpunkt der Kamera bildet eine Basislinie.

Die virtuelle Projektionsebene mit den regelmäßig angeordneten Linienscharen wird auf das Objekt projiziert, wobei die Lage der Kreuzungspunkte durch die Topografie des Objekts paralaktisch verschoben wird. Ein als Target ausgewählter Kreuzungspunkt der Linienscharen auf dem Objekt spannt mit der Basislinie eine Ebene auf, die die virtuelle Projektionsebene in einer Epipolarlinie schneidet. Durch geeignete Justierung der Kamera kann erreicht werden, daß die Basislinie parallel zur Projektionsebene verläuft. Nur dann verlaufen auch die den unterschiedlichen Targets zugehörigen Epipolarlinien in der virtuellen Projektionsebene parallel zueinander.

Auf der Epipolarlinie liegen im allgemeinen mehrere der Kreuzungspunkte der Linienscharen, von denen jedoch nur einer dem Target zuzuordnen ist. Die Mehrdeutigkeit bezüglich der Identifikation des Targets bei der Auswertung des von der Kamera aufgenommenen Bildes kann dadurch verringert werden, daß das Bildfeld und der Arbeitsbereich eingeschränkt werden, der Abstand der Linienscharen vergrößert wird und die Ausrichtung einer Linienschar so gewählt wird, daß die dem Target zugehörige Epipolarlinie geringfügig geneigt zur Linienrichtung verläuft. Bezüglich des Linienabstandes ist zu beachten, daß aufgrund eines grundsätzlich bestehenden Systemmeßfehlers die Epipolarlinien eine Unschärfe aufweisen. Der Neigungswinkel der Epipolarlinien ist daher so zu wählen, daß der Unschärfebereich der Epipolarlinien keine aufeinander folgenden Kreuzungspunkte überdecken kann.

Die Vielzahl der Systemparameter und Einflußgrößen erfordert einen großen Justieraufwand. Eine eindeutige Identifikation der Kreuzungspunkte ist nur innerhalb eines sehr begrenzten Arbeitsvolumens zu erreichen. Die notwendige Justierbarkeit der Projektoren und der Kamera zueinander erfordert einen relativ großen Einbauraum.

Aus US - A - 4 986 262 ist ein Endoskop bekannt, mit dem Unebenheiten auf einem Objekt gemessen werden können. Mit Hilfe eines Laserstrahlenbündels wird ein zweidimensionales Punktemuster auf das Objekt projiziert. Das Punktemuster enthält in einer Spalte dickere Referenzpunkte. Aufgrund von Unebenheiten des Objekts verschieben sich die Referenzpunkte im projizierten Bild gegenüber der Richtung der Referenzspalte und mit ihnen die in derselben Zeile liegenden Meßpunkte. Die Identifikation der Meßpunkte erfolgt durch Abzählen ausgehend von dem Referenzpunkt. Die Zählung versagt, wenn ein oder mehrere Punkte in der Zeile nicht sichtbar sind.

Zur Projektion des Punktemusters wird ein System aus gekreuzten Zylinderlinsen verwendet. Aufgrund des Strahlungsprofils des Laserstrahlenbündels weist das Punktemuster zum Rand hin einen deutlichen Intensitätsabfall auf, wodurch die elektronische Bilderkennung und Bildauswertung erschwert werden.

Aus JP - A - 2002/257 527 ist eine Vorrichtung zur 3-D-Vermessung eines Objekts bekannt, bei der ein Streifenmuster auf das Objekt projiziert wird und das Objekt relativ zum Streifenmuster bewegt wird. Eine senkrecht zur Bewegungsrichtung ausgerichtete Zeilenkamera nimmt einen linearen Bildausschnitt des Objekts und des in diesem Bildausschnitt überlagerten Streifenmusters auf. Das aufgenommene Bild wird einer Bildanalysevorrichtung zur Bestimmung eines Höhenprofils zugeführt. Durch Drehen des Streifenmusters relativ zur Richtung der Zeilenkamera kann die Auflösung der Höhenmessung verändert werden.

Aus DE 196 04 977 A1 ist ein Verfahren zur dreidimensionalen Vermessung dynamischer, d.h. in ihrem Aufbau schnell veränderlicher, Raumbereiche bekannt. Das Verfahren dient der schnellen Vermessung von gefertigten Teilen zur Qualitätskontrolle, zur Ermittlung des geometrischen Aufbaus von Raumbereichen zur Kollisionsvermeidung für Handhabungsgeräte oder zur Ermittlung der Lage und Orientierung bzw. Indentifizierung von Bauteilen oder Baugruppen für die Montage durch Handhabungssysteme. Zum Erreichen einer möglichst kurzen Rechenzeit bei der Raumvermessung werden möglichst viele theoretisch vorbestimmte Koordinatenwerte vorab in indizierten und miteinander verknüpften Tabellen abgelegt. Eine Raumbelegungstabelle enthält die Weltkoordinaten aller bei der entsprechenden geometrischen Anordnung theoretisch möglichen und im Gesichtsfeld einer Kamera befindlichen markanten Raumpunkte. Die markanten Raumpunkte werden durch Projektion eines Signalmusters nach dem Funktionsprinzip eines Dia-Projektors erzeugt. Das Signalmuster muß ausreichend groß sein, um das gesamte Sichtfeld der Kamera durch Zentralprojektion abzudecken. Lichtmusterträger ist eine Scheibe mit kreisförmigen Öffnungen, die durch den Lichtprojektor als ellipsenförmige Flächen auf dem Objekt und im Kamerabild erscheinen.

Der Erfindung lag daher die Aufgabe zugrunde, ein Meßverfahren und ein Meßsystem auf der Grundlage einbelichteter Meßmarken zur parallaktischen Koordinatenbestimmung anzugeben, bei dem eine eindeutige Identifizierung der Meßmarken mit Hilfe eines Bildauswertesystems allein aus der Lage der Meßmarken im Bild des Meßobjekts erfolgen kann und eine der Form des Meßobjekts angepaßte Ausrichtung des Meßsystems nicht erforderlich ist.

Diese Aufgabe wird bei einem Verfahren der eingangs genannten Art erfindungsgemäß durch die kennzeichnenden Merkmale des Anspruchs 1 gelöst. Vorteilhafte Weiterbildungen ergeben sich aus den Merkmalen der Unteransprüche 2 bis 5.

Ein zur Lösung der Aufgabe geeignetes Endoskop der eingangs genannten Art weist erfindungsgemäß die kennzeichnenden Merkmale des Anspruchs 6 auf. Vorteilhafte Ausgestaltungen dazu ergeben sich aus den Unteransprüchen 7 bis 25.

Die grundlegende Erkenntnis der Erfindung besteht darin, daß die erste Achse für die Ausrichtung der Elemente des Meßmusters um einen Winkel $\alpha$ gegenüber der senkrechten Projektion der Basislinie in eine Referenzebene geneigt ist. Die Basislinie ist dabei die Verbindungslinie zwischen den Pupillenmittelpunkten des Projektions- und des Bildaufnahmesystems. Die Referenzebene liegt senkrecht zur optischen Achse des Bildaufnahmesystems im Objektraum in einem Referenzabstand vom Distalende des optischen Systems entfernt. Es ist die Ebene, in der das Meßmuster mit den später näher erläuterten Systemparametern $\alpha$, a, s, t, n, m definiert wird. Durch die Festlegung des Referenzabstandes erhalten die abstandsabhängigen Parameter s und t einen festen Wert. Als Referenzabstand kann z.B. der mittlere Arbeitsabstand gewählt werden, für den das optische System ausgelegt ist.

Bei einem eindimensionalen Meßmuster stellt allein die Neigung unter einem beliebigen Winkel $\alpha \neq 0°$ sicher daß sich bei der parallaktischen Verschiebung der Elemente des Meßmusters jedes Element auf seiner eigenen Verschiebungslinie parallel zur Projektion der Basislinie bewegt. Für einen eindeutigen Nachweis jedes Elementes im Bild müssen jedoch die Größe des Elementes und das Auflösungsvermögen des Bildaufnahmesystems bei der Auswahl des Winkels $\alpha$ berücksichtigt werden. Bei einem zu kleinen Winkel $\alpha$ könnten sonst benachbarte Elemente bei großen lokalen Höhenunterschieden im Meßobjekt aufgrund der daraus folgenden stark unterschiedlichen parallaktischen Verschiebung teilweise zur Überdeckung kommen. Der Winkel $\alpha$ ist daher so zu wählen, daß der Abstand a zwischen den Verschiebelinien jedes Elementes so groß ist, daß für die Fläche jedes Elementes auch bei Regelabweichung der Position und bei einer evtl. gekrümmten Verschiebelinie genügend Raum zum benachbarten Element verbleibt.

Bei einem zweidimensionalen Meßmuster kann die zweite Achse für die Ausrichtung der weiteren Elemente unter einem beliebigen Winkel $\beta$ zur ersten Achse geneigt sein. Aber auch hier ist selbstverständlich darauf zu achten, daß bei der parallaktischen Verschiebung der Gitterelemente keine gegenseitige Überdeckung der Verschiebungslinien entstehen kann. In Abhängigkeit von der Anzahl n und dem Abstand s der Elemente entlang der ersten Achsenrichtung und dem Abstand t der Elemente entlang der zweiten Achsenrichtung hat sich als optimaler Winkel $\alpha = \arctan t \cdot n^{-1} \cdot s^{-1}$ ergeben. Dem entspricht ein Abstand a der Verschiebungslinien in der Größe von $a = t.s \, (n^2 \cdot s^2 + t^2)^{-1/2} \cdot \sin \beta$. Im Folgenden wird der Einfachheit halber meist ein rechter Winkel zwischen den beiden Achsen angenommen.

Bei der durch die Parameter s und t definierten regelmäßigen Anordnung der Elemente des Meßmusters ist zu beachten, daß Fertigungstoleranzen des erzeugenden optischen Bauteils und Abbildungsfehler des Projektionssystems Verzeichnungen des Meßmusters in der Referenzebene bewirken können, die zu leicht gekrümmten Verbindungslinien bei den Achsenrichtungen, Zeilenlinien und Spaltenlinien der Elemente führen. In der Referenzebene können daher die Elemente des Meßmusters um eine Unsicherheit u von der vorgegebenen Regelmäßigkeit abweichen. Um die erfindungsgemäße Indentifizierbarkeit der Elemente des Meßmusters sicherzustellen, sollte die Unsicherheit u kleiner als der halbe Abstand a der Verschiebungslinien des regelmäßigen Meßmusters sein. Es sollte daher gelten $u < 1/2 \cdot t \cdot s \cdot (n^2 \cdot s^2 + t^2)^{-1/2}$. Bei Einhaltung dieser Bedingung haben die Verschiebungslinien trotz einer Verzeichnung keine gemeinsamen Punkte.

Zur Erzeugung des Meßmusters wird zweckmäßigerweise ein diffraktives optisches Element (DOE) verwendet, das mit einem Diodenlaser beleuchtet wird. Ein DOE arbeitet relativ verlustarm und läßt sich bei der Montage im optischen System zur Erzeugung der notwendigen Ausrichtung des projizierten Meßmusters einfach handhaben. Bei einem Meßmuster mit punktförmigen Elementen lassen sich diese mit hoher Intensität und scharfer Bündelung erzeugen.

Die vorgegebene Begrenzung der Anzahl der Elemente längs jeder Achsenrichtung stellt sicher, daß alle in der Bildebene dargestellten Elemente vollständig identifiziert werden können. Die Lage der identifizierten Elemente kann bei der Bildverarbeitung mit den entsprechenden Positionen in Kalibrationsbildern verglichen werden. Evtl. fehlende Elemente haben auf die Identifizierbarkeit der übrigen Elemente keinen Einfluß.

Aus den durch Bildverarbeitung ermittelten Bildkoordinaten des Elementes im Bild des Meßobjektes können die zugehörigen Raumkoordinaten des Elementes im Meßmuster auf dem Meßobjekt ermittelt werden. Die mit ihren Raumkoordinaten identifizierten Elemente können dann als Stützstellen zur Berechnung einer daran angepaßten Oberfläche verwendet werden. Mathematische Methoden zur Berechnung gefitteter Oberflächen sind an sich bekannt und erlauben insbesondere auch eine Korrelation mit entsprechenden Datensätzen aus anderweitig gewonnenen Oberflächenformen.

In der Zeichnung sind Ausführungsbeispiele der Erfindung schematisch dargestellt und werden anhand der Figuren näher beschrieben. Dabei zeigen

Fig.1    die parallaktische Verschiebung eines Punktemusters bei kleiner Abstandsänderung einer ebenen Fläche,

Fig.2    die parallaktische Verschiebung eines Punktemusters zwischen einer ebenen Fläche und einem Körper mit großen Höhenunterschieden,

Fig.3    die parallaktische Verschiebung bei erfindungsgemäßer Anordnung eines Punktemusters,

Fig.4    die geometrischen Bedingungen für die Anordnung des erfindungsgemäßen Punktemusters in einer Referenzebene,

Fig.5    ein Projektionssystem zur Erzeugung des Punktemusters,

Fig.6    einen Aufbau für ein Meßsystem,

Fig.7    einen Schnitt durch die von proximal betrachtete Bildebene des Meßsystems und

Fig.8    einen Schnitt durch die Bildebene bei anderer Anordnung des Projektionssystems.

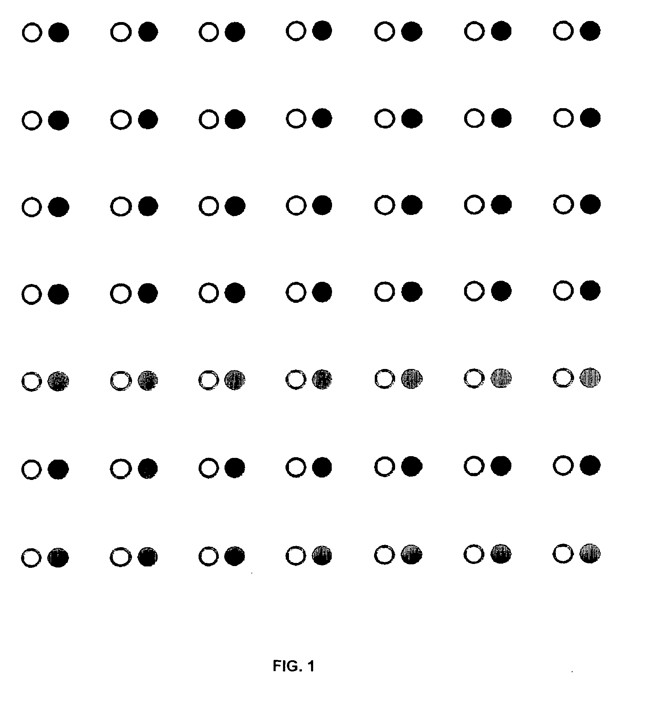

Fig.1 erläutert das Prinzip der parallaktischen Verschiebung eines Punktemusters. Die ausgefüllten Punkte stellen die Lage des Punktemusters auf einem Kalibrationsobjekt in einer bestimmten Projektionsebene dar. Wenn sich der Abstand zu dieser Ebene nur geringfügig verändert, beschreibt das Punktemuster im Bild in erster Näherung eine Seitwärtsverschiebung parallel zu einer Verbindungslinie zwischen den Mittelpunkten der Austrittspupille eines Projektionssystems und der Eintrittspupille eines Bildaufnahmesystems.

Diese Verbindungslinie bildet die Basis des Meßsystems. Bei einer geringen Abstandsänderung ist auch die parallaktische Verschiebung des Punktemusters gering, wie durch die als Ringe dargestellten Punkte deutlich wird. In diesem Fall kann jeder Ring seiner Ausgangslage eindeutig zugeordnet werden.

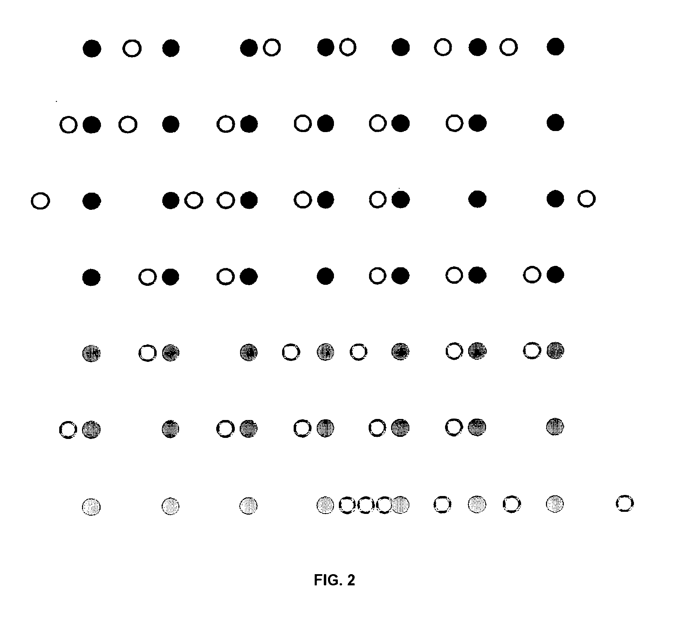

Fig.2 zeigt die parallaktische Verschiebung des Punktemusters bei Projektion auf ein Meßobjekt mit einer Topographie, die deutlich oberhalb und unterhalb der Ebene des Kalibrationsobjektes liegt. Die ausgefüllten Punkte stellen wiederum die Lage des Punktemusters auf dem Kalibrationsobjekt in einer bestimmten Projektionsentfernung dar.

Die als Ringe dargestellten Punkte erscheinen im Bild des Meßobjektes und sind erkennbar keiner bestimmten Ausgangslage zuzuordnen. Eine eindeutige Bestimmung der Raumkoordinaten der einbelichteten Punkte ist nicht möglich, vor allem, wenn auch noch einzelne Punkte im Bild fehlen.

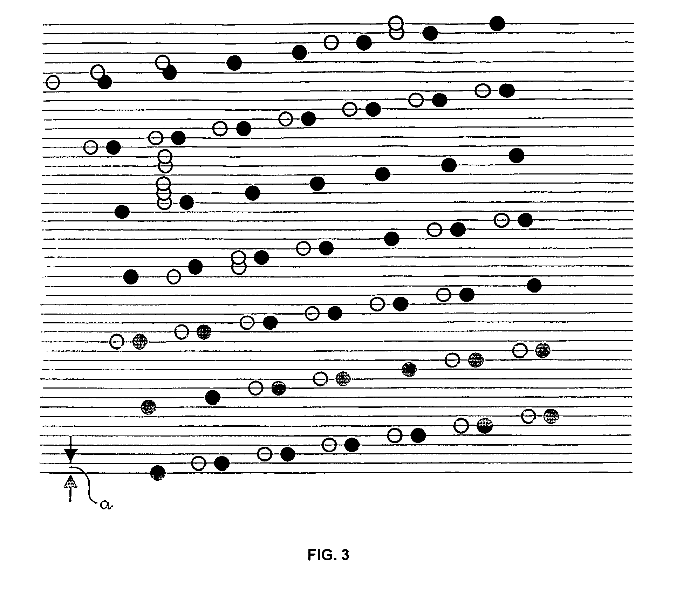

Fig.3 zeigt ein Punktemuster in der erfindungsgemäßen Ausrichtung.

Die dargestellten Linien verlaufen parallel zur Basis des Meßsystems.

Die Achsenrichtungen für das Punktemuster verlaufen unter einem Winkel zu diesen Linien. Dabei ergibt sich ein Abstand a zwischen den Linien, bei dem jeder der ausgefüllten Punkte bei seiner parallaktischen Verschiebung entlang einer eigenen Linie verläuft, auf der keine weiteren Punkte liegen. Die als Ringe dargestellten parallaktisch verschobenen Punkte sind auch bei teilweiser Überdeckung noch getrennt erkennbar und ihrer jeweiligen Ausgangslage zuzuordnen.

Auch fehlende Punkte im parallaktisch verschobenen Bild stören die Auswertung der übrigen Punkte nicht.

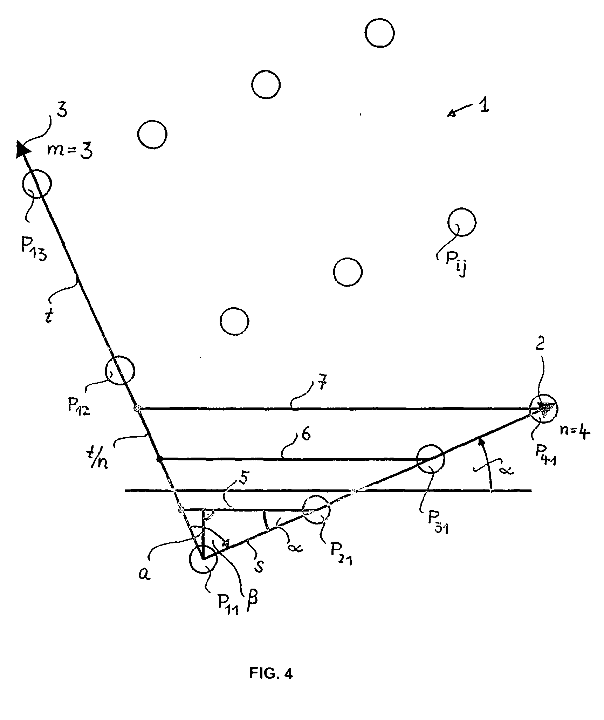

Fig.4 zeigt ein aus Elementen $P_{ij}$ (i = 1, ..., n; j = 1, ..., m) bestehendes Meßmuster 1 auf eine Referenzebene projiziert. In einer ersten Achsenrichtung 2 sind n = 4 Elemente in regelmäßigem Abstand s zueinander angeordnet. In einer zweiten Achsenrichtung 3 sind m = 3 Elemente in regelmäßigem Abstand t zueinander angeordnet. Die Achsenrichtung 2 ist gegenüber der senkrechten Projektion der Basis 4 um einen Winkel $\alpha$ verdreht. Die Achsenrichtung 3 steht in diesem Beispiel senkrecht auf der Achsenrichtung 2, d.h. $\beta = 90°$. Der Winkel $\alpha$ ist so gewählt, daß auf den parallel zur Basis 4 verlaufenden Verschiebungslinien 5, 6, 7 der Elemente $P_{21}$, $P_{31}$, $P_{41}$ keine weiteren Elemente $P_{ij}$ liegen. Der zugehörige Abstand a zwischen den Verschiebungslinien 5, 6, 7 ergibt sich zu $a = t \cdot s \ (n^2 \cdot s^2 + t^2)^{-1/2} \cdot \sin \beta$. Der optimale Winkel $\alpha$ beträgt $\alpha = \arctan t \cdot n^{-1} \cdot s^{-1}$, wobei t/n die Größe der Achsenabschnitte auf der Achsenrichtung 3 ist, die durch die Schnittpunkte der Verschiebungslinien 5, 6, 7 mit der Achsenrichtung 3 entstehen. Eine eindeutige Identifizierung der Elemente $P_{ij}$ im Bild des Meßobjektes ist damit gewährleistet. Der dargestellte Sachverhalt bezieht sich auf ein ideales System. In der Praxis kann das Projektionssystem 8 Verzeichnungen bezüglich der Lage der $P_{ij}$ bewirken.

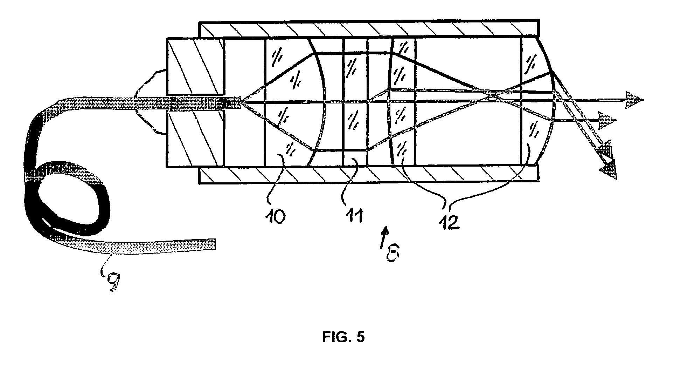

In Fig.5 ist ein Projektionssystem 8 zur Erzeugung des Meßmusters 1 dargestellt. Das Projektionssystem 8 wird von einer Glasfaser 9 mit Licht versorgt. Das Licht gelangt durch eine z.B. asphärische Kollimationslinse 10. Nach der Kollimation besteht das Licht aus ebenen Wellen, die durch ein Diffraktives Optisches Element (DOE) 11 gehen. Dieses spaltet den einen kollimierten, einfallenden Strahl in viele Teilstrahlen auf, die dann das bereits beschriebene Meßmuster mit den Elementen $P_{ij}$ ergeben. Solche DOE sind kommerziell erhältlich und können auch für unterschiedliche Elementformen angefertigt werden. Neben dem Punktemuster kann z.B. auch ein Muster aus gekreuzten Koordinatenlinien geeignet sein, wobei sich dann die Kreuzungspunkte auf unabhängigen Verschiebungslinien bewegen.

Grüne für die Wahl des regelmäßigen Punktemusters sind die kommerzielle Verfügbarkeit des DOE, die gute Erkennbarkeit des Musters, da die Lichtleistung auf kleine Punkte konzentriert ist und die auch bei einer optischen Verzeichnung immer noch relativ runde Form der Punkte, die von einem automatischen Bildverarbeitungsprogramm leicht erkannt werden kann. Das System ist grundsätzlich auch mit unregelmäßigen Meßmustern anwendbar, solange jeder Punkt seine eigene Verschiebungslinie hat.

Nach dem DOE kann ein Linsensystem 12 angeordnet sein, welches den Ausleuchtwinkel des Punktemusters an das Gesichtsfeld eines Bildaufnahmesystems anpaßt.

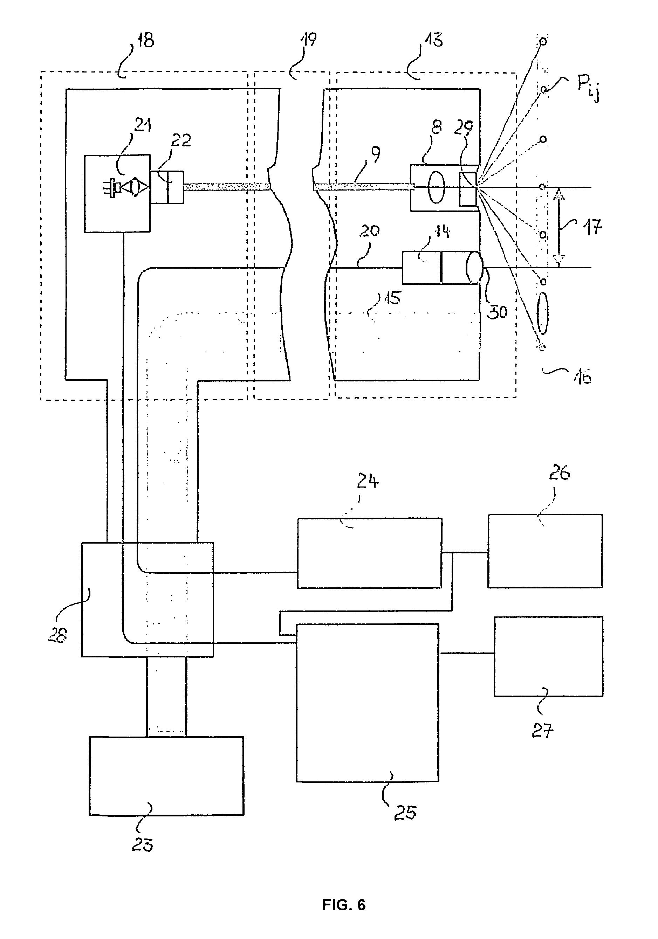

In Fig.6 sind die Komponenten eines vollständigen Meßsystems dargestellt, in das das erfindungsgemäße optische System integriert ist. Das Meßsystem weist einen Kopfteil 13 auf, der ein Projektionssystem 8, ein Bildaufnahmesystem 14 und ein Lichtleitkabel 15 zur Beleuchtung des Meßobjektes enthält. Der Kopfteil kann auch das distale Ende eines Videoendoskops bilden. Die Verbindungslinie zwischen den Pupillenmittelpunkten des Projektionssystems 8 und des Bildaufnahmesystems 14 bildet eine Meßbasis 17. Das Projektionssystem 8 projiziert ein Punktemuster $P_{ij}$ auf die Oberfläche 16 des Meßobjektes.

Der Kopfteil 13 ist mit dem Bedienteil 18 des Meßsystems über einen Schaft 19 verbunden. Der Schaft 19 kann auch ein starres oder flexibles Endoskoprohr sein. In ihm werden die Faser 9 zur Beleuchtung des Projektionssystems 8, die elektrischen Übertragungsleitungen 20 von und zum Bildaufnahmesystem 14, sowie ein Lichtleiterkabel 15 gehalten.

Die Glasfaser 9 wird im Bedienteil 18 von einer Laserdiode 21 gespeist. Der Fasertyp kann je nach den Anforderungen multimodal, singlemodal oder singlemodal-polarisationserhaltend gewählt werden. Zur besseren Montierbarkeit ist eine lösbare Steckverbindung 22 vorgesehen.

Die Funktion des im Projektionssystem 8 enthaltenen DOE basiert auf wellenlängenabhängigen Beugungseffekten. Die Größe des Meßmusters ändert sich daher in erster Näherung proportional mit der Wellenlänge. Um die daraus resultierende Meßunsicherheit zu eliminieren, kann entweder die Wellenlänge durch Thermostatisierung der Laserdiode 21 konstant gehalten werden, oder es wird die Wellenlänge über eine Temperaturmessung an der Laserdiode 21 erfaßt, um die Größenänderung des Meßmusters rechnerisch zu kompensieren.

Das soweit beschriebene Meßsystem ist mit mehreren Konsolengeräten verbunden. Eine Kaltlichtquelle 23 liefert z.B. die Lichtenergie zur Beleuchtung des Meßobjektes über das Lichtleiterkabel 15. Als Bildaufnahmesystem 14 ist z.B. eine Videokamera mit CCD-Chip vorgesehen, die über einen Kamerakontroller 24 gesteuert wird. Ein Computer 25 steuert über seine Software im wesentlichen die Bildaufnahme, die Bildverwaltung, die Bildverarbeitung, die Kalibration des Meßsystems, die Ausführung der Messung, die Steuerung der Laserdiode 21 und die Temperaturmessung und -Stabilisierung. Dem Kamerakontroller 24 und dem Computer 25 können ein erster und ein zweiter Monitor 26, 27 zugeordnet sein. Der Anschluß der Konsolengeräte an das Bedienteil 18 kann über einen Steckverteiler 28 erfolgen.

Zur Gewinnung geeigneter Meßwerte wird die Laserdiode 21 zweckmäßigerweise in rascher Folge bildsynchron ein- und ausgeschaltet. Dies erlaubt zum einen der Bildverarbeitungssoftware die in der Technik übliche Differenzbildung zwischen aufeinanderfolgenden Bildern mit und ohne Meßmuster. Die Differenz enthält dann weitgehend nur noch die Bildinformation des Meßmusters. Ferner ist dann auch ein Bild ohne das störende Meßmuster für Beobachtungszwecke vorhanden.

Die Laserdiode 21 kann außerdem in der Intensität noch mit einem Rauschen moduliert werden, wodurch eine leichte Variation der Wellenlänge und damit einhergehend eine Fluktuation der Specklestruktur hervorgerufen wird. Das verbessert die Positionsbestimmung der Elemente des Meßmusters und damit die Meßgenauigkeit des Systems.

Das Projektionssystem 8 ist in der dargestellten Schnittebene neben dem Bildaufnahmesystem 14 angeordnet. Bei der Montage wird es leicht rotiert, so daß die Orientierung des Meßmusters in dem bereits beschriebenen Maß leicht schräg zur Projektion der Basis 17 liegt. Dies hat bei der Bilddarstellung den optischen Effekt eines schiefen Meßmusters zur Folge.

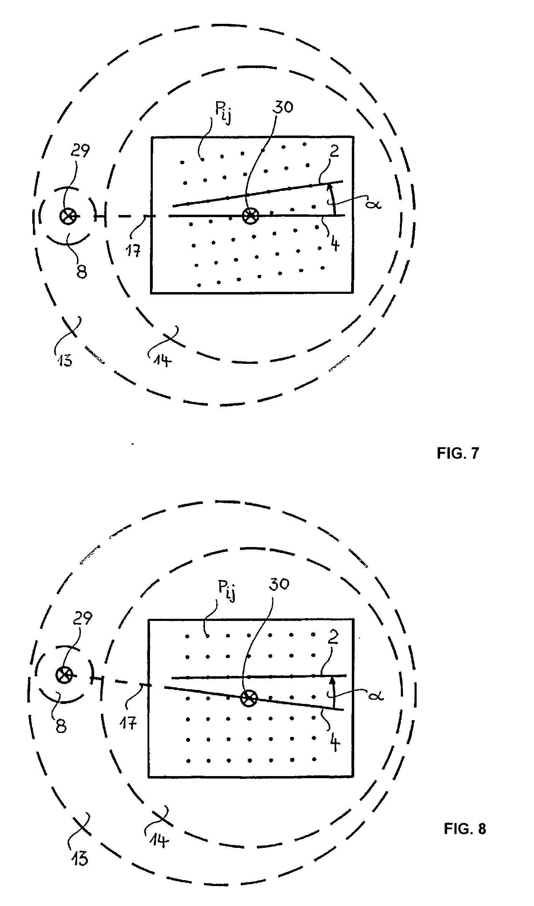

Fig.7 zeigt den Schnitt durch die Bildebene in der Aufsicht mit dem projizierten Punktemuster $P_{ij}$ und der senkrechten Projektion 4 der Meßbasis 17. Die Meßbasis 17 ist in der Darstellung die Verbindungslinie zwischen dem Mittelpunkt 29 der Austrittspupille des Projektionssystems 8 und dem Mittelpunkt 30 Eintrittspupille des Bildaufnahmesystems 14.

Es ist jedoch auch möglich, das Projektionssystem 8 so anzuordnen, daß die Projektion 4 der Meßbasis schiefwinklig zu einer Bildkante verläuft. In Fig.8 ist dieser Spezialfall dargestellt, bei dem die Projektion der Meßbasis den Windel $\alpha$ mit einer Bildkante bildet. Dies bewirkt, daß die erste Achsenrichtung 2 parallel zu einer Bildkante ausgerichtet ist, was einer gebräuchlicheren Ansicht entspricht. Dabei verlaufen aber die Verschiebungslinien schiefwinklig zu der Bildkante.

Die Auswertung des parallaktisch verschobenen Meßmusters im Bild des Meßobjektes kann grundsätzlich nach zwei verschiedenen Methoden erfolgen.

Die Grundlage eines analytischen Verfahrens besteht darin, für jedes Meßsystem werksseitig mit Hilfe von Testbildern Kalibrationsparameter zu bestimmen und abzuspeichern. Die Parameter sind: Bildkoordinaten aller Elemente $P_{ij}$ bei dem gewählten Kalibrationsabstand, die Basis, die Brennweite, die Verzeichnung und der individuelle Zentrierfehler der Optik.

Bei der Messung muß das Meßsystem die Elemente $P_{ij}$ identifizieren, d.h. die Indizes i, j müssen eindeutig bestimmt werden. Mit an sich bekannten mathematischen Formeln werden aus den jeweiligen Bildkoordinaten u, v auf dem CCD-Chip der Kamera die Raumkoordinaten x, y, z errechnet.

Grundlage eines Interpolationsverfahrens ist die Aufnahme von n Kalibrationsbildern in n Kalibrationsabständen, wobei ein Koordinatengitter als Kalibrationsobjekt verwendet wird. Im Kalibrationsbild k werden die Elemente $P(k)_{ij}$ identifiziert. Für jedes Element $P(k)_{ij}$ werden die Bildkoordinaten $u(k)_{ij}$ und $v(k)_{ij}$ erfaßt und mit Hilfe der Koordinatenlinien auf dem Kalibrationsobjekt die Raumkoordinaten $x(k)_{ij}$, $y(k)_{ij}$, $z(k)_{ij}$ ermittelt, wobei z(k) dem jeweiligen Kalibrationsabstand entspricht, der für alle Elemente in der k-ten Kalibrationsebene gleich ist. Für alle $x_{ij}$ wird mit den n Kalibrationswerten ein Polynom (n-1)-ten Grades in $u_{ij}$ gebildet und ebenso für $y_{ij}$ und $z_{ij}$, womit der einmalig durchzuführende Kalibrationsprozeß abgeschlossen ist.

Bei der Messung werden für jeden Punkt die Indizes i, j und die Bildkoordinaten $u_{ij}$ erkannt. Mit den gefundenen $u_{ij}$ werden aus den Kalibrations-Polynomen die Raumkoordinaten x, y, z aller Elemente berechnet, in die dann eine Fläche gefittet werden kann, welche die tatsächliche Oberfläche des Meßobjektes annähert. In dieser nachgebildeten Fläche können anschließend durch Superposition mit dem Bild des Meßobjektes sichtbare Merkmale, wie z.B. Schadstellen, Risse oder Korrosionsflächen, bezeichnet und in ihrer Größe vermessen werden.

**Bezugszeichenliste**

| | |
|---|---|
| 1 | Meßmuster |
| 2 | erste Achsenrichtung |
| 3 | zweite Achsenrichtung |
| 4 | senkrechte Projektion der Basis |
| 5 | Verschiebungslinie |
| 6 | Verschiebungslinie |
| 7 | Verschiebungslinie |
| 8 | Projektionssystem |
| 9 | Glasfaser |
| 10 | Kollimationslinse |
| 11 | Diffraktives Optisches Element (DOE) |
| 12 | Linsensystem |
| 13 | Kopfteil |
| 14 | Bildaufnahmesystem |
| 15 | Lichtleitkabel |
| 16 | Oberfläche Meßobjekt |
| 17 | Meßbasis |
| 18 | Bedienteil |
| 19 | Schaft |
| 20 | Übertragungsleitung |
| 21 | Laserdiode |
| 22 | Steckverbindung |
| 23 | Kaltlichtquelle |
| 24 | Kamerakontroller |
| 25 | Computer |
| 26 | erster Monitor |
| 27 | zweiter Monitor |
| 28 | Steckverteiler |
| 29 | Mittelpunkt der Austrittspupille Projektionssystem |
| 30 | Mittelpunkt der Eintrittspupille Bildaufnahmesystem |

**Patentansprüche**

**1.** Verfahren zur endoskopischen Vermessung der Topographie eines Meßobjekts mit Projektion eines aus regelmäßig angeordneten Elementen bestehenden Meßmusters auf die Oberfläche eines Meßobjekts, mit Aufnahme eines Bildes des Meßobjekts mit aufprojiziertem Meßmuster durch eine zur Projektion entlang einer Meßbasis beabstandete Kamera und mit einer Auswertung der aufgrund der Topographie des Meßobjekts parallaktisch verschobenen Lage der Elemente des Meßmusters im Bild des Meßobjekts, wobei die Elemente des Meßmusters längs mindestens einer ersten, zur Meßbasis geneigt verlaufenden Achsenrichtung so angeordnet werden, daß sich jedes Element bei parallaktischer Verschiebung auf einer eigenen, zur senkrechten Projektion der Meßbasis parallel verlaufenden Verschiebungslinie bewegt, die mit anderen Verschiebungslinien keine gemeinsamen Punkte besitzt und aus der Position auf seiner Verschiebungslinie die Raumkoordinaten eines zugehörigen Punktes des Meßobjekts bestimmt werden, **dadurch gekennzeichnet, daß**

a) das vollständige Meßmuster aus einer beschränkten Anzahl von lokal begrenzten, durch Laserprojektion in einer Referenzebene erzeugten Elementen gebildet wird, wobei die Referenzebene senkrecht zur optischen Achse der Kamera im Objektraum in einem Referenzabstand vom Projektionssystem gewählt wird,
b) die Systemparameter des Meßmusters aus den in die Referenzebene projizierten Elementen ermittelt werden,
c) den Elementen in der Referenzebene und in dazu unterschiedlich beabstandeten Pojektionsebenen Kalibrationsparameter zugeordnet und in einem Speicher abgelegt werden,
d) zur Vermessung der Topographie die einzelnen Elemente hinsichtlich ihrer Anordnung innerhalb des Meßmusters identifiziert werden und aus ihrer Lage im Bild des Meßmusters unter Berücksichtigung der Kalibrationsparameter die zugehörigen Raumkoordinaten errechnet werden.

2. Verfahren nach Anspruch 1, **dadurch gekennzeichnet, daß** alternativ zu den Schritten c) und d) eine Kalibrierung durchgeführt wird, bei der die Lage der Elemente des Meßmusters mit ihren Raumkoordinaten bei unterschiedlichen Abständen zu einem Kalibrationsobjekt mit Koordinatengitter aufgenommen und abgespeichert wird, so daß bei der Vermessung eines Meßobjekts nach Identifizierung des Elements des Meßmusters und Bestimmung seiner Lage im Bild des Meßobjekts die zugehörigen Raumkoordinaten aus einem Speicher abgerufen werden können.

3. Verfahren nach Anspruch 2, **dadurch gekennzeichnet, daß** die Verschiebungslinie, die zur Identifizierung eines Elements des Meßmusters berücksichtigt werden kann und aus der bei der aktuellen Messung auch Zwischenwerte der Verschiebung entnommen werden können, aus den bei der Kalibrierung ermittelten diskreten Raumkoordinaten errechnet wird.

4. Verfahren nach Anspruch 1, **dadurch gekennzeichnet, daß** zu den ermittelten Raumkoordinaten mit Hilfe eines mathematischen Algorithmus die Parameter einer passenden Raumfläche ermittelt werden, in der Merkmale räumlich vermessen werden können, nachdem sie in der Superposition mit dem Bild des Meßobjekts markiert wurden.

5. Verfahren nach Anspruch 4, **dadurch gekennzeichnet, daß** insbesondere bei Anwendungen in der medizinischen Endoskopie die ermittelten Raumflächen mit entsprechenden Raumflächen korreliert werden, die für dasselbe Meßobjekt mit anderen Meßmethoden, wie z.B. Ultraschall, Computertomographie, Magnetoresonanz, gewonnen wurden.

6. Endoskop zur Vermessung der Topographie eines Meßobjekts mit einem Projektionssystem (8) zur Projektion eines optisch erkennbaren Meßmusters (1) auf die Oberfläche (16) eines zu vermessenden Objektbereichs und einem Bildaufnahmesystem (14) und Bildauswertungssystem zur Ermittlung der parallaktisch verschobenen Bildkoordinaten des Meßmusters (1) im zu vermessenden Objektbereich, wobei der Abstand der Pupillenmittelpunkte (29, 30) des Projektionssystems (8) und des Bildaufnahmesystems (14) eine Meßbasis (17) bildet und die Elemente des Meßmusters (1) in zumindest einer ersten Achsenrichtung (2) regelmäßig angeordnet sind und diese Achsenrichtung (2) gegenüber der senkrechten Projektion (4) der Meßbasis (17) um einen Winkel $\alpha$ verdreht ist, bei dem sich die Elemente ($P_{ij}$) des Meßmusters (1) bei einer Änderung des Projektionsabstandes im Bild des Meßobjekts auf zur senkrechten Projektion der Meßbasis (17) parallelen Verschiebungslinien bewegen, die keine gemeinsamen Punkte haben, **dadurch gekennzeichnet, daß** das vollständige Meßmuster aus einer beschränkten Anzahl von lokal begrenzten, durch Laserprojektion erzeugten Elementen ($P_{ij}$) besteht, die hinsichtlich ihrer Anordnung innerhalb des projizierten vollständigen Meßmusters (1) eindeutig indentifizierbar sind, und eine elektronische Recheneinheit (Computer) (25) mit Speicher vorhanden ist, die zu den identifizierten Elementen die in einer Referenzebene zugeordneten Systemparameter und die in unterschiedlich beabstandeten Projektionsebenen zugeordneten Kalibrationsparameter ermittelt und daraus die zugehörigen Raumkoordinaten des Meßobjektes errechnet.

7. Endoskop nach Anspruch 6, **dadurch gekennzeichnet, daß** ein zweidimensionales Meßmuster (1) vorgesehen ist, bei dem eine begrenzte Anzahl weiterer lokal begrenzter Elemente ($P_{ij}$) in einer zweiten Achsenrichtung (3) und der durch beide Achsenrichtungen (2, 3) aufgespannten Ebene regelmäßig angeordnet sind, wobei die zweite Achsenrichtung (3) zur ersten Achsenrichtung (2) einen beliebigen Winkel $0 < \beta < 180°$ bildet.

8. Endoskop nach Anspruch 7, **dadurch gekennzeichnet, daß** die Elemente ($P_{ij}$) des Meßmusters (1) eine n·m-Matrix bilden mit n Spalten und m Zeilen, wobei die n Spalten entlang der ersten Achsenrichtung (2) jeweils um s und die m Zeilen entlang der zweiten Achsenrichtung (3) jeweils um t beabstandet sind.

9. Endoskop nach Anspruch 6, **dadurch gekennzeichnet, daß** das Meßmuster (1) aus punktförmigen und/oder ring-förmigen Elementen ($P_{ij}$) besteht.

10. Endoskop nach Anspruch 6, **dadurch gekennzeichnet, daß** das Meßmuster (1) Elemente aus gekreuzten Linien aufweist.

11. Endoskop nach Anspruch 6, **dadurch gekennzeichnet, daß** für den Abstand a der Verschiebungslinien (5, 6, 7) gilt:

$$a = t \cdot s \cdot (n^2 \cdot s^2 + t^2)^{-1/2} \cdot \sin \beta.$$

12. Endoskop nach Anspruch 7, **dadurch gekennzeichnet, daß** für Abweichungen des projizierten Meßmusters (1) von der regelmäßigen Anordnung seiner Elemente ($P_{ij}$) in einer Referenzebene eine Unsicherheit u < 1/2·a gilt.

13. Endoskop nach Anspruch 6, **dadurch gekennzeichnet, daß** zur Erzeugung des Meßmusters (1) im Projektionssystem (8) ein Diffraktives Optisches Element (DOE) (11) vorgesehen ist.

14. Endoskop nach Anspruch 13, **dadurch gekennzeichnet, daß** das Projektionssystem (8) justierbar angeordnet ist.

15. Endoskop nach Anspruch 13, **dadurch gekennzeichnet, daß** eine dem Projektionssystem (8) zugeordnete Lichtquelle zur Beleuchtung des DOE (11) in der Helligkeit und/oder Wellenlänge modulierbar ist.

16. Endoskop nach Anspruch 15, **dadurch gekennzeichnet, daß** als Lichtquelle ein Laser vorgesehen ist.

17. Endoskop nach Anspruch 16, **dadurch gekennzeichnet, daß** als Laser eine Laserdiode (21) vorgesehen ist.

18. Endoskop nach Anspruch 17, **dadurch gekennzeichnet, daß** der Laserdiode (21) eine Temperaturmeßeinrichtung zugeordnet ist.

19. Endoskop nach Anspruch 18, **dadurch gekennzeichnet, daß** die Temperaturmeßeinrichtung mit einer Vorrichtung zur Temperaturstabilisierung der Laserdiode (21) gekoppelt ist.

20. Endoskop nach Anspruch 6, **dadurch gekennzeichnet, daß** als Bildaufnahmesystem (14) eine Videokamera vorgesehen ist.

21. Endoskop nach Anspruch 6, **dadurch gekennzeichnet, daß** eine Kaltlichtquelle (23) zur Ausleuchtung (31) des Meßobjektes vorgesehen ist.

22. Endoskop nach Anspruch 6, **dadurch gekennzeichnet, daß** eine Steuereinrichtung zur alternierenden Bildaufnahme mit und ohne aufprojiziertem Meßmuster (1) vorgesehen ist.

23. Endoskop nach Anspruch 6, **dadurch gekennzeichnet, daß** zur Steuerung, Bildspeicherung und Bildauswertung ein Rechner (25) mit Bildauswertesoftware vorgesehen ist.

24. Endoskop nach einem der Ansprüche 6 bis 23, **dadurch gekennzeichnet, daß** das Bildaufnahmesystem (14) und das Projektionssystem (8) am distalen Ende des Endoskops angeordnet sind, wobei die dem Projektionssystem (8) zugeordnete Lichtquelle (21) am proximalen Ende des Endoskops angeordnet ist und zur Lichtübertragung eine Glasfaser (9) vorgesehen ist.

25. Endoskop nach Anspruch 6, **dadurch gekennzeichnet, daß** die senkrechte Projektion (4) der Meßbasis (17) um den Winkel $\alpha$ gegenüber einer Bildkante des Bildaufnahmesystems (14) gedreht ist.

**EP 1 597 539 B1**

**Claims**

1. Method for endoscopic measuring of the topography of a test object, the method comprising a measurement pattern consisting of regularly arranged elements being projected onto the surface of a test object, comprising an image of the test object with a measurement pattern projected thereon being recorded using a camera that is at a distance along a measuring base for projection purposes, and comprising the position of the elements of the measurement pattern being evaluated in the image of the test object, the position of the elements of the measurement pattern having been displaced parallactically due to the topography of the test object, in which the elements of the measurement pattern are arranged along at least a first axial direction running at an angle to the measuring base such that each element moves on an individual displacement line running parallel to the vertical projection of the measuring base in the case of a parallactic displacement, which displacement line does not have any common points with other displacement lines, and the spatial coordinates of an associated point of the test object are determined from the position of the element along the displacement line thereof, **characterized in that**

   a) the complete measurement pattern is formed from a limited number of locally bounded elements generated by laser projection in a reference plane, the reference plane being selected to be perpendicular to the optical axis of the camera in the object space at a reference distance from the projection system,
   b) the system parameters of the measurement pattern are determined from the elements projected into the reference plane,
   c) calibration parameters are associated with the elements in the reference plane and in projection planes at different distances therefrom, and said parameters are stored in a storage medium,
   d) the individual elements are identified in respect of their arrangement within the measurement pattern for measuring the topography, and the associated spatial coordinates are calculated from the position of said elements in the image of the measurement pattern taking into account the calibration parameters.

2. Method according to Claim 1, **characterized in that**, as an alternative to steps c) and d), a calibration is performed in which the position of the elements of the measurement pattern is recorded using a coordinate grid, with the spatial coordinates of the elements being at different distances from a calibration object, and said position is stored, and so the associated spatial coordinates can be recalled from a storage medium during the measurement of a test object after the element of the measurement pattern has been identified and its position in the image of the test object has been determined.

3. Method according to Claim 2, **characterized in that** the displacement line is calculated from the discrete spatial coordinates determined during the calibration, which displacement line can be taken into account for identifying an element of the measurement pattern and from which displacement line intermediate values of the displacement can also be gathered during the current measurement.

4. Method according to Claim 1, **characterized in that** the parameters of a fitting three-dimensional surface are determined for the determined spatial coordinates with the aid of a mathematical algorithm, on which three-dimensional surface features can be measured in space after they have been marked in the superposition with the image of the test object.

5. Method according to Claim 4, **characterized in that**, particularly in medical endoscopy applications, the determined three-dimensional surfaces are correlated to corresponding three-dimensional surfaces obtained for the same test object using other measurement methods, such as ultrasound, computed tomography or magnetic resonance imaging.

6. Endoscope for measuring the topography of a test object, the endoscope comprising a projection system (8) for projecting an optically visible measurement pattern (1) onto the surface (16) of an object region to be measured, and an image recording system (14) and image evaluation system for determining the parallactically displaced image coordinates of the measurement pattern (1) in the object region to be measured, in which the distance between the pupil centre points (29, 30) of the projection system (8) and the image recording system (14) forms a measuring base (17) and the elements of the measurement pattern (1) are arranged regularly in at least a first axial direction (2) and this axial direction (2) is twisted by an angle $\alpha$ compared to the perpendicular projection (4) of the measuring base (17), at which angle the elements ($P_{ij}$) of the measurement pattern (1) move along displacement lines that have no common points and are parallel to the perpendicular projection of the measuring base (17) when the projection distance in the image of the test object is changed, **characterized in that** the complete measurement pattern consists of a limited number of locally bounded elements ($P_{ij}$) generated by laser projection, which elements can be unambiguously identified in respect of their arrangement within the projected complete measurement pattern (1), and there is an electronic computational unit (computer) (25) with a storage medium, which for the identified elements determines the associated system parameters in a reference plane and the associated calibration parameters in projection planes at different distances and which thereby calculates the associated spatial coordinates of the test object.

7. Endoscope according to Claim 6, **characterized in that** provision is made for a two-dimensional measurement pattern (1), in which a limited number of additional locally bounded elements ($P_{ij}$) are arranged regularly in a second axial direction (3) and in the plane spanned by both axial directions (2, 3), in which the second axial direction (3) forms an arbitrary angle $0 < \beta < 180°$ with respect to the first axial direction (2).

8. Endoscope according to Claim 7, **characterized in that** the elements ($P_{ij}$) of the measurement pattern (1) form an n-m matrix with n columns and m rows, in which the n columns are respectively spaced apart along the first axial direction (2) by s and the m rows are respectively spaced apart along the second axial direction (3) by t.

9. Endoscope according to Claim 6, **characterized in that** the measurement pattern (1) consists of punctiform and/or annular elements ($P_{ij}$).

10. Endoscope according to Claim 6, **characterized in that** the measurement pattern (1) has elements made of crossed lines.

11. Endoscope according to Claim 6, **characterized in that** the following holds true for the spacing a between the displacement lines (5, 6, 7):

$$a = t \cdot s \cdot (n^2 \cdot s^2 + t^2)^{-1/2} \cdot \sin \beta.$$

12. Endoscope according to Claim 7, **characterized in that** an uncertainty of $u < 1/2 \cdot a$ holds true for deviations of the projected measurement pattern (1) from the regular arrangement of its elements ($P_{ij}$) in a reference plane.

13. Endoscope according to Claim 6, **characterized in that** a diffractive optical element (DOE) (11) is provided for generating the measurement pattern (1) in the projection system (8).

14. Endoscope according to Claim 13, **characterized in that** the projection system (8) is arranged in an adjustable fashion.

15. Endoscope according to Claim 13, **characterized in that** a light source associated with the projection system (8) for illuminating the DOE (11) can be modulated in respect of the brightness and/or the wavelength.

16. Endoscope according to Claim 15, **characterized in that** a laser is provided as a light source.

17. Endoscope according to Claim 16, **characterized in that** a laser diode (21) is provided as a laser.

18. Endoscope according to Claim 17, **characterized in that** a temperature measurement apparatus is associated with the laser diode (21).

19. Endoscope according to Claim 18, **characterized in that** the temperature measurement apparatus is coupled to a device for stabilizing the temperature of the laser diode (21).

20. Endoscope according to Claim 6, **characterized in that** a video camera is provided as an image recording system (14).

21. Endoscope according to Claim 6, **characterized in that** a cold light source (23) is provided for illuminating (31) the test object.

22. Endoscope according to Claim 6, **characterized in that** a control apparatus is provided for alternating image recording with and without a measurement pattern (1) projected thereon.

**23.** Endoscope according to Claim 6, **characterized in that** a computer (25) with image evaluation software is provided for control, image storage and image evaluation.

**24.** Endoscope according to one of Claims 6 to 23, **characterized in that** the image recording system (14) and the projection system (8) are arranged at the distal end of the endoscope, with the light source (21) associated with the projection system (8) being arranged at the proximal end of the endoscope and provision being made for an optical fibre (9) for transmitting light.

**25.** Endoscope according to Claim 6, **characterized in that** the perpendicular projection (4) of the measuring base (17) is rotated by the angle $\alpha$ compared to an image edge of the image recording system (14).


**Revendications**

**1.** Procédé de mesure par endoscopie de la topographie d'un objet à mesurer, avec :

projection d'un motif de mesure constitué d'éléments disposés régulièrement à la surface d'un objet à mesurer, enregistrement d'une image de l'objet à mesurer sur laquelle est projeté le motif de mesure, par une caméra située à une distance de la projection qui correspond à la base de mesure et

évaluation de la position déplacée par parallaxe suite à la topographie de l'objet à mesurer des éléments du motif de mesure dans l'image de l'objet à mesurer,

les éléments du motif de mesure étant disposés dans au moins une première direction axiale qui s'étend obliquement par rapport à la base de mesure, de telle sorte que par déplacement de parallaxe, chaque élément se déplace sur une ligne de déplacement propre qui s'étend parallèlement à la projection perpendiculaire de la base de mesure et qui ne possède pas de point commun avec d'autres lignes de déplacement,

les coordonnées spatiales d'un objet associé de l'objet à mesurer étant déterminées à partir de sa position sur sa ligne de déplacement,

**caractérisé en ce que**

a) le motif complet de mesure est formé d'un nombre limité d'éléments délimités localement et formés par projection laser dans un plan de référence, le plan de référence sélectionné étant perpendiculaire à l'axe optique de la caméra dans l'espace d'objet et à une distance de référence du système de projection,

b) les paramètres systémiques du motif de mesure sont déterminés à partir des éléments projetés dans le plan de référence,

c) des paramètres d'étalonnage sont associés aux éléments présents dans le plan de référence et dans des plans de projection situés à différentes distances de ce dernier et sont conservés dans une mémoire,

d) pour mesurer la topographie, l'agencement des différents éléments à l'intérieur du motif de mesure est identifié et les coordonnées spatiales qui leur associées sont calculées à partir de leur position dans l'image du motif de mesure en tenant compte des paramètres d'étalonnage.

**2.** Procédé selon la revendication 1, **caractérisé en ce qu'**en variante aux étapes c) et d), il exécute un étalonnage dans lequel la position des éléments du motif de mesure est enregistrée avec leurs coordonnées spatiales à différentes distances d'un objet d'étalonnage doté d'une grille de coordonnées et est conservée en mémoire, de sorte que lors de la mesure d'un objet à mesurer, les coordonnées spatiales associées peuvent être reprises dans une mémoire après identification de l'élément de motif de mesure et détermination de sa position dans l'image de l'objet à mesurer.

**3.** Procédé selon la revendication 2, **caractérisé en ce que** la ligne de déplacement qui peut être prise en compte pour identifier un élément du motif de mesure et à partir de laquelle des valeurs intermédiaires du déplacement peuvent être obtenues lors de la mesure en cours est calculée à partir des coordonnées spatiales discontinues et déterminées lors de l'étalonnage.

**4.** Procédé selon la revendication 1, **caractérisé en ce que** les paramètres d'une surface spatiale qui s'ajustent aux coordonnées spatiales déterminées sont déterminés à l'aide d'un algorithme mathématique, cette surface spatiale permettant de mesurer spatialement les caractéristiques après qu'elles ont été repérées dans la superposition sur l'image de l'objet à mesurer.

**5.** Procédé selon la revendication 4, **caractérisé en ce qu'**en particulier dans les applications en endoscopie médicale, les surfaces spatiales déterminées sont mises en corrélation avec des surfaces spatiales correspondantes qui ont été obtenues pour le même objet à mesurer à l'aide d'autres méthodes de mesure, par exemple les ultrasons, la tomographie assistée par ordinateur ou la résonance magnétique.

**6.** Endoscope destiné à mesurer la topographie d'un objet à mesurer et présentant :

un système de projection (8) qui projette un motif de mesure (1) reconnaissable visuellement sur la surface (16) d'une partie à mesurer de l'objet et
un système (14) d'enregistrement d'image ainsi qu'un système d'évaluation d'image qui détermine les coordonnées d'image déplacées par parallaxe du motif de mesure (1) dans la partie de l'objet à mesurer,
la distance entre les centres de pupille (29, 30) du système de projection (8) et du système (14) d'enregistrement d'image formant une base de mesure (17) et les éléments du motif de mesure (1) étant disposés régulièrement dans au moins une première direction axiale (2), cette direction axiale (2) étant tournée d'un angle $\alpha$ par rapport à la projection perpendiculaire (4) de la base de mesure (17),
les éléments ($P_{ij}$) du motif de mesure (1) se déplaçant dans l'image de l'objet sur des lignes de déplacement de mesure parallèles à la projection perpendiculaire de la base de mesure (17), ces lignes de déplacement ne présentant pas de points communs, **caractérisé en ce que**
le motif de mesure complet est constitué d'un nombre limité d'éléments ($P_{ij}$) délimités localement et formés par projection laser, dont l'agencement à l'intérieur du motif de mesure complet (1) projeté peut être identifié de manière univoque et
**en ce qu'**une unité électronique de calcul (ordinateur) (25) dotée d'une mémoire est prévue pour déterminer pour les éléments identifiés les paramètres systémiques associés à un plan de référence et les paramètres d'étalonnage associés dans des plans de projection situés à distances différentes pour en calculer les coordonnées spatiales associées de l'objet à mesurer.

**7.** Endoscope selon la revendication 6, **caractérisé en ce qu'**il présente un motif de mesure (1) en deux dimensions dans lequel un nombre limité d'autres éléments ($P_{ij}$) limités localement sont disposés de manière régulière dans une deuxième direction axiale (3) et dans le plan sous-tendu par les deux directions axiales (2, 3), la deuxième direction axiale (3) formant avec la première direction axiale (2) un angle quelconque $0 < \beta < 180°$.

**8.** Endoscope selon la revendication 7, **caractérisé en ce que** les éléments ($P_{ij}$) du motif de mesure (1) forment une matrice n-m qui compte n colonnes et m lignes, les n colonnes étant disposées à une distance mutuelle s dans la première direction axiale (2) et les m lignes à une distance mutuelle t dans la deuxième direction axiale (3).

**9.** Endoscope selon la revendication 6, **caractérisé en ce que** le motif de mesure (1) est constitué d'éléments ($P_{ij}$) ponctuels et/ou annulaires.

**10.** Endoscope selon la revendication 6, **caractérisé en ce que** le motif de mesure (1) présente des éléments constitués de lignes croisées.

**11.** Endoscope selon la revendication 6, **caractérisé en ce que** la distance a entre les lignes de déplacement (5, 6, 7) est régie par la relation

$$a = t.s.(n^2.s^2 + t^2)^{-1/2}.\sin \beta.$$

**12.** Endoscope selon la revendication 7, **caractérisé en ce qu'**en cas d'écart entre le motif de mesure (1) projeté par rapport à l'agencement régulier de ces éléments ($P_{ij}$) dans un plan de référence, l'incertitude u est < 1/2.a.

**13.** Endoscope selon la revendication 6, **caractérisé en ce qu'**un élément optique diffractif (DOE) (11) est prévu pour former le motif de mesure (1) dans le système de projection (8).

**14.** Endoscope selon la revendication 13, **caractérisé en ce que** le système de projection (8) est ajustable.

**15.** Endoscope selon la revendication 13, **caractérisé en ce qu'**une source de lumière associée au système de projection (8) et servant à éclairer le DOE (11) peut être modulée en luminosité et/ou en longueur d'onde.

**EP 1 597 539 B1**

**16.** Endoscope selon la revendication 15, **caractérisé en ce qu'**un laser est prévu comme source de lumière.

**17.** Endoscope selon la revendication 16, **caractérisé en ce qu'**une diode laser (21) est prévue comme laser.

**18.** Endoscope selon la revendication 17, **caractérisé en ce qu'**un dispositif de mesure de température est associé à la diode laser (21).

**19.** Endoscope selon la revendication 18, **caractérisé en ce que** le dispositif de mesure de température est couplé à un dispositif de stabilisation de la température de la diode laser (21).

**20.** Endoscope selon la revendication 6, **caractérisé en ce qu'**une caméra vidéo est prévue comme système (14) d'enregistrement d'image.

**21.** Endoscope selon la revendication 6, **caractérisé en ce qu'**une source de lumière froide (23) est prévue pour éclairer (31) l'objet à mesurer.

**22.** Endoscope selon la revendication 6, **caractérisé en ce qu'**il présente un dispositif de commande qui alterne l'enregistrement d'images avec et sans projection d'un motif de mesure (1).

**23.** Endoscope selon la revendication 6, **caractérisé en ce qu'**un calculateur (25) doté d'un logiciel d'évaluation d'image est prévu pour la commande, l'enregistrement des images et l'évaluation des images.

**24.** Endoscope selon l'une des revendications 6 à 23, **caractérisé en ce que** le système (14) d'enregistrement d'images et le système de projection (8) sont disposés à l'extrémité distale de l'endoscope, la source de lumière (21) associée au système de projection (8) étant disposée à l'extrémité proximale de l'endoscope et une fibre de verre (9) étant prévue pour la transmission de la lumière.

**25.** Endoscope selon la revendication 6, **caractérisé en ce que** la projection perpendiculaire (4) de la base de mesure (17) est tournée d'un angle $\alpha$ par rapport à un bord de l'image du système (14) d'enregistrement d'image.

FIG. 1

FIG. 2

FIG. 3

FIG. 4

FIG. 5

EP 1 597 539 B1

FIG. 6

20

FIG. 7

FIG. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5150254 A1
- US 5669871 A1
- DE 3516164 C2
- WO 9303579 A
- US 4986262 A
- JP 2002257527 A
- DE 19604977 A1

**In der Beschreibung aufgeführte Nicht-Patentliteratur**

- *Machine Vision News,* 1999, vol. 4